# EUROPEAN PATENT APPLICATION

(11) **EP 4 640 247 A1**
(43) Date of publication of application: **29.10.2025**
(21) Application number: 23907885.0
(22) Date of filing: 22.12.2023
(51) Int. Cl.: A61L 27/10, A61L 27/56, C04B 28/34, C04B 18/24, C04B 22/00, C04B 24/06, C04B 38/02, A61C 8/02

(54) **SLURRY FOR LOW-TEMPERATURE CURING OF CERAMIC POROUS BODY, AND METHOD FOR MANUFACTURING CERAMIC POROUS BODY**

(30) Priority: 22.12.2022 KR 20220182062
(71) Applicant: Biotree Co., Ltd., Gyeongsan-si, Gyeongsangbuk-do 38541 (KR)
(72) Inventor: KIM, Sukyoung, Gyeongsan-si Gyeongsangbuk-do 38541 (KR); KIM, Jooseong, Sangju-si, Gyeongsangbuk-do 37204 (KR)
(74) Representative: Marks & Clerk LLP
(86) International application number: PCT/KR2023/021470
(87) International publication number: WO 2024/136602

(57) **Abstract**

The present invention provides slurry for low-temperature curing a ceramic porous body including a foaming agent, a binder, cement powder, and cellulose fiber. According to the slurry for low-temperature curing the ceramic porous body and a method for manufacturing a ceramic porous body using the same, a ceramic porous body retaining biocompatibility and osteoconductivity may be manufactured through low-temperature curing, and the final cured porous body may also exhibit high mechanical strength due to the formation of stabilized pores during foaming in a pore formation operation.

## Description

### [Technical Field]

The present invention relates to slurry for low-temperature curing a ceramic porous body and a method for manufacturing a ceramic porous body by using the same, and particularly, to an eco-friendly slurry for low-temperature curing a ceramic porous body that manufactures a ceramic porous body only with a cement curing reaction without a high-temperature sintering process, and a method for manufacturing a ceramic porous body using the same.

### [Background Art]

Porous ceramics refers to solids in which pores of various sizes are distributed in lumps, granules, or particles, and are also called porous bodies, porous solids, or porous materials.

In general, the pore size of the artificially manufactured ceramic porous body varies from 1 µm to 10 mm, and the shape also varies, and these characteristics vary depending on the manufacturing method.

Depending on the presence or absence of heat treatment, the ceramic porous body may be divided into a sintered ceramic porous body and a non-sintered ceramic porous body, and the most common ceramic porous body is a sintered type and may be broadly divided into a particle aggregate type, a sponge type or a foam type, and a honeycomb type.

The ceramic porous body is used in various applications, such as a filtration or diffusion filter, a medium catalyst body, a sound absorber, a DPF, a heat exchanger, a special heater, a bone graft material, or a scaffold, depending on the material and size and shape of the pores of the ceramic porous body. Various pore forming agents are used as shown in the table below to form pores of various sizes in the ceramic porous body.

A general method of manufacturing a ceramic porous body is to form a ceramic porous body by mixing a certain amount of flux material with ceramic particles controlled to the desired size and melting the flux by a high-temperature treatment to agglomerate ceramic particles, or to obtain a porous body by compressing and forming ceramic particles of the desired particle size and then partially sintering the formed ceramic particles at a temperature lower than the sintering temperature.

The pores are generated from the internal micro-pores present in the particles and the macro-pores present between the particles, and the size of the pore present between the particles is related to the size of the raw material particle. These methods not only make it difficult to effectively control the pore size, distribution, and the like of the porous body, but also make it difficult to increase the porosity to 50% or more.

On the other hand, bone graft materials or scaffolds require a porous body that exhibits biomimetic, biodegradability, porous structures interconnected in three dimensions, appropriate mechanical properties, bone formation induction, osteoconductivity, and the like to induce more efficient bone tissue regeneration than existing porous bodies.

The existing ceramic porous body manufacturing method, the coral or sponge replication method, and the sacrificial casting method using polymers and salts as molds, were mainly used. In recent years, the development of 3D printing technology has enabled a porous body with a three-dimensional pore structure, pore size, shape, and porosity, but due to the high-temperature sintering process, new bone regeneration was significantly lower than the generation of homogeneous or heterogeneous bones due to a decrease in biocompatibility and osteoconductivity.

In addition, with the development of 3D printing technology and low-temperature process technology, it is possible to manufacture a porous body with excellent biocompatibility, but the technology of direct foaming using ceramic slurry and low-temperature curing technology are far more economical and simple than the 3D printing technology. However, the problem in the technology combining the direct foaming method and the low-temperature curing process at this point is that it is necessary to stabilize the pores formed during foaming and mechanical strength is low after sintering. To overcome these problems, first, it is necessary to improve the shape of the wet porous body and stabilization of the pores when foaming the ceramic slurry, and second, a technology to increase the mechanical strength of the final porous body through a cement reaction at room temperature instead of the existing high-temperature sintering or high-temperature and high-pressure.

Accordingly, due to global environmental regulations and the demand for excellent biocompatible products, rather than a manufacturing method including a traditional high-temperature sintering process, a technology for slurry for low-temperature curing a ceramic porous body that may manufacture a ceramic porous body maintaining biocompatibility and osteoconductivity through low-temperature curing rather than a high-temperature process, and form stabilized pores during foaming in a pore forming operation and a method for manufacturing a ceramic porous body using the same has been required.

### [Prior Art Literature]

### [Patent Document]

(Patent Document 0001) Korean Patent Application Publication No. 10-2016-0113594 (September 30, 2016)

### [Technical Problem]

The present invention has been made in an effort to provide slurry for low-temperature curing a ceramic porous body that may manufacture a ceramic porous body of which biocompatibility and osteoconductivity are maintained through low-temperature curing and form stabilized pores during foaming in a pore forming operation, and a method for manufacturing a ceramic porous body by using the same.

The problem to be solved by the present invention is not limited to the above-mentioned problems, and the problems not mentioned will be clearly understood by those skilled in the art from the present specification and the accompanying drawings.

### [Technical Solution]

A first aspect of the present invention for achieving the object provides slurry for low-temperature curing of a ceramic porous body, the slurry containing a foaming agent, a binder, cement powder, and cellulose fiber.

Preferably, the foaming agent may include at least one of propyl gallate, butyl gallate, hexylamine, butyric acid, valeric acid, lauryl betaine, and coco-betaine.

Preferably, the binder may be a hydrogel including one or more selected from alginate, carrageenan, agar, PVA, PVP, and PEO.

Preferably, the cement powder may be cement powder of one selected from apartite cement, brushite cement, octacalcium phosphate (OCP) cement, and Portland cement.

A second aspect of the present invention for achieving the object provides a method of manufacturing a ceramic porous body, the method including: a slurry preparing operation of preparing slurry by mixing cement powder and a solution containing a binder; A foaming agent adding operation of adding a foaming agent to the slurry; a ceramic porous body low-temperature curing slurry preparing operation of preparing slurry for low-temperature curing a ceramic porous body by adding cellulose fiber to the slurry to which the foaming agent is added; a foaming operation of forming pores by stirring the slurry for low-temperature curing the ceramic porous body; a preliminary porous body manufacturing operation of manufacturing a preliminary porous body by pouring the slurry for low-temperature curing the ceramic porous body that has undergone the foaming operation into a mold and then curing cement; a crosslinking operation of crosslinking the preliminary porous body to improve mechanical strength; and a drying operation of drying the crosslinked preliminary porous body.

Preferably, the crosslinking operation may be performed by immersing the preliminary porous body in a solution containing a divalent or trivalent cation.

Preferably, the preliminary porous body manufacturing operation may be performed at 35 to 80°C.

Preferably, the drying operation may be performed at 35 to 50°C.

### [Advantageous Effects]

The slurry for low-temperature curing the ceramic porous body and the method for manufacturing a ceramic porous body using the same according to the present invention may manufacture a ceramic porous body retaining biocompatibility and osteoconductivity through low-temperature curing, and form stabilized pores during foaming in a pore formation operation.

### [Description of Drawings]

FIG. 1 is a diagram illustrating the formation of pores and changes in the shape of a porous body in accordance with the amount of binder added at the constant P/L ratio (=0.6) according to an embodiment of the present invention.
FIG. 2 is a diagram illustrating comparison of the pore shape and the porous body fracture surface according to the amount of foaming agent added at the constant P/L ratio (=0.6) and the amount of binder (=1.0 wt%) according to an embodiment of the present invention.
FIG. 3 is a photograph illustrating a change in characteristics of an OCP porous body depending on a P/L ratio in slurry with a constant binder content (alginate 1.0 wt%) and a constant foaming agent content (coco betaine 0.5 wt%) according to an embodiment of the present invention.
FIG. 4 is a photograph illustrating a change in the characteristics of an OCP porous body depending on the foaming agent content in the slurry with a constant binder content (alginate 1.0 wt%) and a constant P/L ratio according to an embodiment of the present invention.

### [Best Mode]

The above-described and additional aspects are embodied through embodiments described with reference to the accompanying drawings. It is understood that the components of each embodiment are possible in various combinations with the components of the embodiment or other embodiments as long as there is no other mention or contradiction between them. Based on the principle that the inventor can appropriately define the concept of the term to describe his or her invention in the best way, the terms used in this specification and claims should be construed as meanings and concepts consistent with the content described or the proposed technical idea.

Hereinafter, exemplary embodiments of the present invention will be described in detail with reference to the accompanying drawings. However, the present invention is not limited to the embodiments described herein and may be embodied in other forms. Rather, the embodiments introduced herein are provided so that the disclosed content may be thorough and complete, and the spirit of the present invention may be sufficiently transmitted to those skilled in the art. Throughout the specification, the same reference numerals represent the same components, and components denoted by the same or similar reference numerals perform the same or similar functions, and thus descriptions thereof may be omitted. For components having the same reference numerals of which the description is omitted, the above description may be referred to for components having the same or similar reference numerals.

According to the first aspect of the present invention for achieving the object, the present invention provides slurry for low-temperature curing a ceramic porous body, which includes a foaming agent, a binder, cement powder, and cellulose fiber.

The slurry includes the foaming agent to form stable pores.

The slurry includes the binder, thereby increasing pore formation and stability, and also increasing mechanical strength.

The slurry includes the ceramic porous body, so that the ceramic porous body may be manufactured by a cement curing reaction and manufactured at a low temperature without a sintering operation.

The slurry includes the cellulose fiber, so that it is possible to increase the shape and stabilization of the pores of the ceramic porous body manufactured. Specifically, the low mechanical strength and pore stability of the wet porous body, which were a problem when the existing ceramic slurry was directly foamed, were solved by adding cellulose in a relatively simple method, and the result of improving the mechanical strength and pore stability in the cemented porous body was obtained.

Preferably, the foaming agent may include at least one of propyl gallate, butyl gallate, hexylamine, butyric acid, valeric acid, lauryl betaine, and coco betaine.

Since the foaming agent includes at least one of propyl gallate, butyl gallate, hexylamine, butyric acid, valeric acid, lauryl betaine, and coco betaine, most of the foaming agents have amphiphilic properties through a surface treatment process for changing the surface of cement particles from hydrophilic to hydrophobic, and the hydrophilic portion of the foaming agent adheres to the surface of the hydrophilic cement particles to form a layer, thereby helping to form stable pores by allowing the cement particles to be hydrophobic from the outside. Any foaming agent may be used as the foaming agent used in this case, but coco betaine was used as the foaming agent in the present invention.

The amount of coco betaine binder added is preferably 0.2 to 0.7 parts by weight based on 100 parts by weight of the binder solution, and most preferably, the case where the amount of coco betaine binder is 0.5 parts by weight is the most effective. When the amount of foaming agent added was less than 0.2 parts by weight, only a part of the cement particles became hydrophobic, and pore formation was low, and when the amount of foaming agent exceeded 0.7 parts by weight, there were many foaming agents that did not adhere to the cement particles, which significantly reduced the stability of the formed porous body, resulting in cracking or collapse after drying.

Preferably, the binder may be a hydrogel including one or more selected from alginate, carrageenan, agar, PVA, PVP, and PEO.

The binder is a hydrogel including one or more selected from alginate, carrageenan, agar, PVA, PVP, and PEO, and may improve pore formation or stability of a wet porous body or a porous body after low-temperature curing, and may increase mechanical strength.

In the present invention, a binder solution was prepared by adding Na-alginate as a binder additive at a weight ratio of 1.0% to distilled water. The amount of Na-alginate added was possible by 0.5 to 2.0% based on the weight of the cement powder, but preferably 1% by weight was most preferable. In this case, when Na-alginate was not added or when Na-alginate was added at the content lower than 1%, pore formation or stability was significantly reduced, resulting in cracking after drying, and ultimately, the collapse of the porous body occurred due to low strength.

In addition, when Na-alginate is added more than the weight ratio of 2.0%, there is a concern that osteoconductivity or biocompatibility may be lowered due to a large amount of Na-alginate. In the present embodiment, the binder solution was added to the OCP cement powder (α-TCP+NaH₂PO₄, see Korean Patent Application No. 10-2021-0157523) by P/L=0.6 and then mixed. The mixing weight ratio (P/L ratio) of the cement powder and the binder solution was slightly different depending on the type of the powder, but when the alginate solution was mixed with the OCP cement powder, the mixing weight ratio was 0.2 to 0.9, preferably 0.5 to 0.7, and the mixing weight ratio of 0.6 was the most effective.

Preferably, the cement powder may be cement powder of one selected from apartite cement, brushite cement, octacalcium phosphate (OCP) cement, and Portland cement. The cement powder refers to a compound excluding H₂O among the main raw materials and reactants, which are not final materials in Table 1 below.

**[Table 1]**

| Type | Calcium phosphate cement | | | | |
|---|---|---|---|---|---|
| | Apatite cement | | Brushite cement | OCP cement | Portland cement (PC) |
| Main raw material | β-TCP | TTC | β-TCP | α-TCP | PC + plaster |
| Reactant | H₂O | DCPA/D CPD+H₂ O | MCPM/PCPA+H₂O | NaH₂PO₄+H₂O | H₂O |
| Final material | CDHA | HA | brushite (DCPD) | OCP | CSH compound |
| HA: Ca₁₀(PO₄)₆(OH)₂ hydroxyapatite; | | | | | |
| CDHA: Ca₁₀-x(PO₄)₆(OH)₂-x calcium deficient hydroxyapatite; | | | | | |
| TCP: Ca₃(PO₄)₂T tricalcium phosphate; OCP: | | | | | |
| OCP:Ca₈H₂(PO₄)_{6.}5H₂O octacalcium phosphate; TTCP: | | | | | |
| TTCP:Ca₄(PO₄)₂O tetracalcium phosphate; | | | | | |
| DCPD: CaHPO₄.2HO₂ dicalcium phosphate dihydrate; | | | | | |
| DCPA: CaHPO₄ dicalcium phosphate anhydrate, | | | | | |

The present invention according to a second aspect for achieving the object provides a method of manufacturing a ceramic porous body, the method including: a slurry preparing operation of preparing slurry by mixing cement powder and a solution containing a binder; a foaming agent adding operation of adding a foaming agent to the slurry; a ceramic porous body low-temperature curing slurry preparing operation of preparing slurry for low-temperature curing a ceramic porous body by adding cellulose fiber to the slurry to which the foaming agent is added; a foaming operation of forming pores by stirring the slurry for low-temperature curing the ceramic porous body; a preliminary porous body manufacturing operation of manufacturing a preliminary porous body by pouring the slurry for low-temperature curing the ceramic porous body that has undergone the foaming operation into a mold and then curing cement; a crosslinking operation of crosslinking the preliminary porous body to improve mechanical strength; and a drying operation of drying the crosslinked preliminary porous body.

Preferably, it may be characterized that the crosslinking operation is performed by immersing the preliminary porous body in a solution containing a divalent or trivalent cation.

The crosslinking operation is performed by immersing the preliminary porous body in a solution containing a divalent or trivalent cation, thereby improving mechanical characteristics of the porous body.

Preferably, it may be characterized that the preliminary porous body manufacturing operation is performed at 35 to 80°C. The preliminary porous body manufacturing operation is performed at 35 to 80°C, so that the cement curing reaction may be performed at a relatively low temperature. As the temperature is increased within the temperature range, the curing reaction occurs faster.

Preferably, it may be characterized that the drying operation is performed at 35 to 50°C. The drying operation is performed at 35 to 50°C to increase the mechanical strength of the porous body cured at the low temperature.

Hereinafter, the present invention will be described in more detail with reference to embodiments. However, the present invention should not be limitedly interpreted by the following embodiments.

### Embodiment 1 - Prepare slurry for low-temperature curing of ceramic porous body

The process of preparing slurry for low-temperature curing a ceramic porous body is as follows.

### (1) Slurry preparing operation of preparing slurry by mixing cement powder and a solution containing a binder

In an operation of mixing cement mixed powder and an aqueous solution containing a binder, an organic binder solution was mixed with cement powder while adjusting a weight (L) ratio of the solution per weight of the cement powder (P) (P/L ratio).

In the present invention, a binder solution was prepared by adding Na-alginate as a binder additive at a weight ratio of 1.0% to distilled water. The amount of Na-alginate added was possible by 0.5 to 2.0% based on the weight of the cement powder, but preferably 1% by weight was most preferable. In this case, when Na-alginate was not added or when Na-alginate was added at the content lower than 1%, pore formation or stability was significantly reduced, resulting in cracking after drying, and ultimately, the collapse of the porous body occurred due to low strength. In addition, when Na-alginate is added more than the weight ratio of 2.0%, there is a concern that osteoconductivity or biocompatibility may be lowered due to a large amount of Na-alginate.

In the present embodiment, the binder solution was added to the OCP cement powder (α-TCP+NaH₂PO₄) by P/L=0.6 and then mixed.

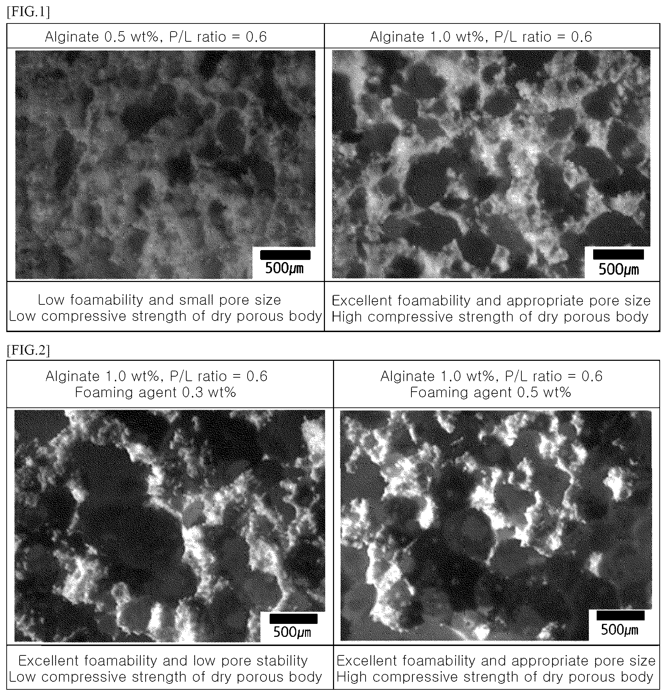

The mixing weight ratio (P/L ratio) of the cement powder and the binder solution was slightly different depending on the type of the powder, but when the alginate solution was mixed with the OCP cement powder, referring to FIG. 1, the mixing weight ratio was 0.2 to 0.9, preferably 0.5 to 0.7, and the mixing weight ratio of 0.6 was the most effective.

### (2) Adding operation of adding a foaming agent to the slurry

Coco betaine was used as a foaming agent. Referring to FIG. 2, the amount of coco betaine binder added is preferably 0.2 to 0.7 parts by weight based on 100 parts by weight of the binder solution, and most preferably 0.5 parts by weight was the most effective. When the amount of foaming agent added was less than 0.2 parts by weight, only some of the cement particles became hydrophobic, and thus pore formation was low, and when the amount of foaming agent exceeded 0.7 parts by weight, there were many foaming agents that did not adhere to the cement particles, which significantly reduced the stability of the formed porous body, resulting in cracking or collapse after drying.

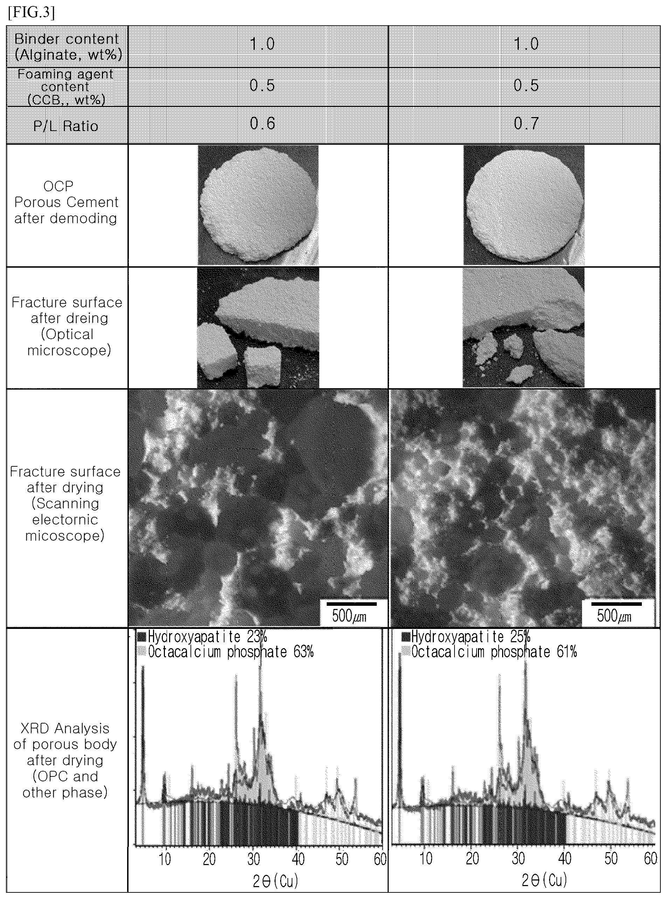

After preparing cement slurry with the solution to which the amounts of the binder and the foaming agent presented above are added, the stable micropore shape of the porous body and the pore structure have excellent drying strength. When the foaming agent content (0.9%) lower than the suggested mixing ratio is added, the strength of the porous body is reduced due to the increase in the size of pores due to high viscosity, so it is important to obtain the desired pore size and porosity through the adjustment of the binder, the foaming agent, and the P/L ratio (see FIG. 3).

(3) Ceramic porous body low-temperature curing slurry preparing operation of preparing slurry for low-temperature curing a ceramic porous body by adding cellulose fiber to the slurry to which the foaming agent is added.

In a cellulose fiber adding operation, commercial micrometer or nanometer-sized cellulose fibers that were well dispersed in advance were added to the slurry. In this case, the amount of cellulose added may vary depending on the cellulose fiber content in the commercial product, but the cellulose fiber content used in the present invention is 0.1 to 4.0 parts by weight, but when it is in the range of 0.2 to 0.4 parts by weight, pore formation was observed without agglomeration between cellulose fibers. When the cellulose fiber content was out of the above range, a phenomenon of pore collapse due to heterogeneous dispersion was observed, and the mechanical strength was rather reduced.

### Embodiment 2 - Manufacture ceramic porous body

### (1) Foaming operation of forming pores by stirring the slurry for low-temperature curing the ceramic porous body

In the stirring and foaming operation, the slurry to which the cellulose was added was uniformly dispersed by using a stirrer at room temperature and a desired pore size was formed. Pores are formed by introducing air into the slurry during stirring, which is the foaming operation of forming pores. The introduced air is hydrophobic and separated from the water, and hydrophobic cement particles adhere around the air bubbles by surface treatment, resulting in the formation of a cement particle layer outside the air bubbles. When pores are formed, the injection pattern of air varies depending on the stirring speed and time, and by using this, the pore size, shape, distribution, and the like may be easily controlled. In the present invention, the slurry was stirred for 2 to 10 minutes to form pores with a size of about 500 to 700 microns. Preferably, it was performed for 4 to 6 minutes, and most preferably for 5 minutes. That is, when the stirring and foaming operation was performed under the above optimized foaming conditions, a ceramic porous body having the stability of the pore shape and the desired size and distribution could be obtained.

### (2) Preliminary porous body manufacturing operation of manufacturing a preliminary porous body by pouring the slurry for low-temperature curing the ceramic porous body that has undergone the foaming operation into a mold and curing cement

The molding and cement reaction operation is a process of pouring the foamed slurry into a desired mold and curing the slurry through a cement reaction in a thermo-hygrostat. In the present invention, in order to perform the cement reaction, the temperature of the thermo-hygrostat was set to 37 to 80°C at a constant relative humidity (95% RT), and the reaction time was 2 to 6 hours. That is, under constant humidity conditions, the reaction temperature section was preferably 37 to 80°C, but the most preferable temperature was 70°C and the reaction time was 2 hours.

### (3) Crosslinking operation of crosslinking the preliminary porous body to improve mechanical strength

In the crosslinking operation, after the cement reaction was completed, the porous body was demolded, and then Na-alginate was immersed in a calcium ion solution to cross-link the Na-alginate in order to increase the strength of the porous body. In the operation of mixing the binder solution with cement powder, 1 wt% Na-alginate is contained in the organic binder solution, and the mechanical properties of the porous body may be improved by immersing the alginate in a multi-ion solution and cross-linking the alginate. In this case, various divalent or trivalent ion solutions may be used, and in the present example, calcium chloride (CaCl₂) or calcium acetate (Ca(C₂H₃O₂)₂) is used as a calcium ion source. Using distilled water, a 1 to 6 wt%, preferably 4 wt%, a solution of calcium acetate was prepared and used. At this concentration, the crosslinking was performed for the crosslinking time of 2 to 10 minutes, preferably 5 minutes at room temperatures. When the calcium ion concentration is too low, sufficient crosslinking is not performed, and when the porous body is sufficiently cross-linked, higher compressive strength than that of the non-crosslinked porous body may be obtained. After crosslinking, the porous body was immersed in distilled water for 24 hours and then washed.

### (4) Drying operation of drying the crosslinked preliminary porous body

In the drying operation, after crosslinking, the washed cement porous body was put in a dryer and dried at 40°C for 24 hours. This cement porous body has a relatively lower mechanical strength than the porous body manufactured by the high-temperature sintering method, but it may be manufactured by a low-temperature process, so it has the advantage of being able to apply a bioactive material or drug. In addition, the cement porous body of the present invention is characterized by excellent biocompatibility and osteoconductivity that may be applied to a bone graft material or scaffold, and mechanically processed.

### Experimental Example

FIG. 1 is a diagram illustrating the formation of pores and changes in the shape of a porous body in accordance with the amount of binder added at a constant P/L ratio (=0.6) as a result according to the embodiment of the present invention. Referring to FIG. 1, the 1.0 wt% aqueous solution showed more excellent pore shape and drying strength when the weight mixing ratio of the alginate aqueous solution and the OCP cement mixed powder (α-TCP and NaH₂PO₄) per weight was P/L=0.6 and the addition of the foaming agent was made constant at 0.5 wt% and the alginate content as a binder in the aqueous solution was changed to 0.5 wt% and 1 wt%.

FIG. 2 is a diagram illustrating comparison of a pore shape and a porous body fracture surface according to the amount of foaming agent added as a result according to the embodiment of the present invention. Referring to FIG. 2, when the foaming agent content was changed to 0.3 wt% and 0.5 wt% at the constant amount of binder (=1.0 wt%) and the constant P/L ratio (=0.6) per weight of the OCP cement mixed powder, 0.5 wt% of the foaming agent showed more excellent pore shape and drying strength.

FIG. 3 is a photograph illustrating a change in characteristics of an OCP porous body depending on a P/L ratio in slurry with the constant binder content (alginate 1.0 wt%) and the constant foaming agent content (coco betaine 0.5 wt%) according to the embodiment of the present invention. Referring to FIG. 3, from the top figure, an optical micrograph of the OCP cement porous body dried after demolding, an optical micrograph and a scanning electron micrograph of the fracture surface of the OCP porous body after curing, and the results of x-ray diffraction analysis (XRD) are in order. The obtained results showed a slight difference according to the P/L ratio. The biggest difference is the pore size and porosity of the OCP porous body, and small pores and low porosity were obtained under conditions of the large P/L ratio or the large amount of liquid phase (0.7), and when the P/L ratio was 0.6, the desired pore size (about 200 to 400 µm) and porosity (>60%) were obtained. Here, it can be seen that the final phase of the OCP porous body cured by the final cement reaction was obtained with OCP (>60%), HA (>20%), and other phases, and it is independent of the preparing conditions of the cement slurry.

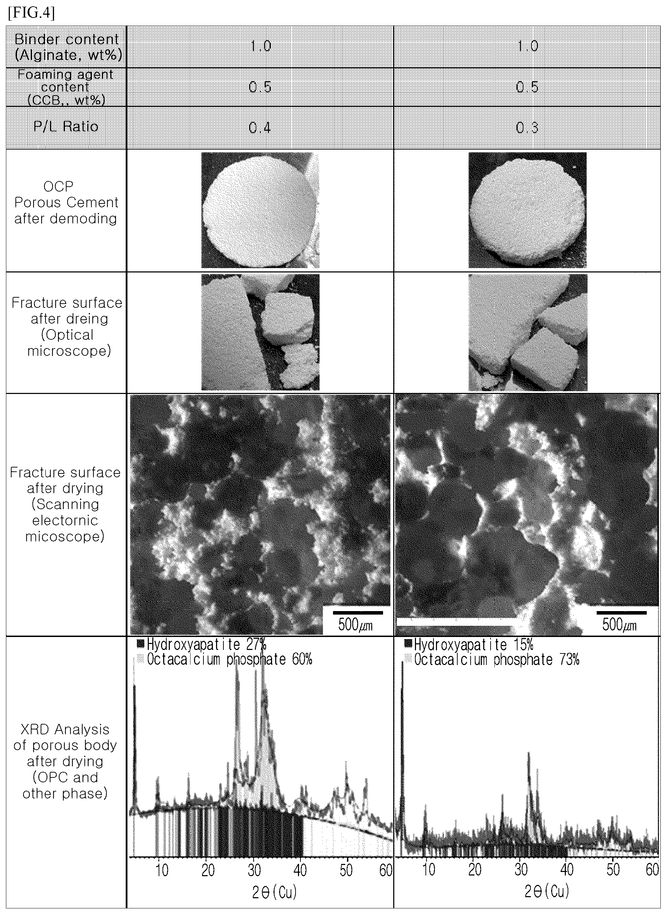

FIG. 4 is a photograph illustrating a change in the characteristics of an OCP porous body depending on the foaming agent content in the slurry with the constant binder content (alginate 1.0 wt%) and the constant P/L ratio according to the embodiment of the present invention. Referring to FIG. 4, from the top figure, an optical micrograph of the OCP cement porous body dried after demolding, an optical micrograph and a scanning electron micrograph of the fracture surface of the OCP porous body after curing, and the results of x-ray diffraction analysis (XRD) are in order. The obtained results showed a slight difference according to the amount of foaming agent added, and the biggest difference was the pore size of the OCP porous body. A stable pore shape was obtained under a condition in which the amount of foaming agent added was 0.4 wt%, but when the amount of foaming agent added was 0.3 wt%, more stable and slightly large pores (about 300 to 500 µm) were obtained. Here, it can be seen that the final phase of the OCP porous body cured by the final cement reaction was obtained with OCP (>60%), HA (>20%), and other phases, and it is independent of the preparing conditions of the cement slurry.

Although the present invention has been described above through embodiments referring to the accompanying drawings, the present invention is not limited thereto, and should be interpreted to cover various modifications that may be clearly derived by those skilled in the art. The patent claims are intended to cover these modifications.

## Claims

1. Slurry for low-temperature curing a ceramic porous body, the slurry containing a foaming agent, a binder, cement powder, and cellulose fiber.

2. The slurry of claim 1, wherein the foaming agent includes at least one of propyl gallate, butyl gallate, hexylamine, butyric acid, valeric acid, lauryl betaine, and coco betaine.

3. The slurry of claim 1, wherein the binder is hydrogel including one or more selected from alginate, carrageenan, agar, PVA, PVP, and PEO.

4. The slurry of claim 1, wherein the cement powder is cement powder of one selected from apartite cement, brushite cement, octacalcium phosphate (OCP) cement, and Portland cement.

5. A method of manufacturing a ceramic porous body, the method comprising:
a slurry preparing operation of preparing slurry by mixing cement powder and a solution containing a binder;
a foaming agent adding operation of adding a foaming agent to the slurry;
a ceramic porous body low-temperature curing slurry preparing operation of preparing slurry for low-temperature curing a ceramic porous body by adding cellulose fiber to the slurry to which the foaming agent is added;
a foaming operation of forming pores by stirring the slurry for low-temperature curing the ceramic porous body;
a preliminary porous body manufacturing operation of manufacturing a preliminary porous body by pouring the slurry for low-temperature curing the ceramic porous body that has undergone the foaming operation into a mold and then curing cement;
a crosslinking operation of crosslinking the preliminary porous body to improve mechanical strength; and
a drying operation of drying the crosslinked preliminary porous body.

6. The method of claim 5, wherein the crosslinking operation is performed by immersing the preliminary porous body in a solution containing a divalent or trivalent cation.

7. The method of claim 5, wherein the preliminary porous body manufacturing operation is performed at 35 to 80°C.

8. The method of claim 5, wherein the drying operation is performed at 35 to 50°C.
